# EUROPEAN PATENT APPLICATION

(11) **EP 3 780 001 A1**
(43) Date of publication of application: **17.02.2021**
(21) Application number: 19776192.7
(22) Date of filing: 27.02.2019
(51) Int. Cl.: G16H 50/30, A61B 5/00, G06Q 50/22

(54) **HEALTH ASSISTANCE SYSTEM, INFORMATION PROVIDING SHEET OUTPUT DEVICE, METHOD, AND PROGRAM**

(30) Priority: 26.03.2018 JP 2018058535
(71) Applicant: NEC Solution Innovators, Ltd., Koto-ku, Tokyo 136-8627 (JP)
(72) Inventor: TANAKA Hirofumi, Tokyo 136-8627 (JP); TAJIRI Toshikazu, Tokyo 136-8627 (JP)
(74) Representative: Betten & Resch
(86) International application number: PCT/JP2019/007647
(87) International publication number: WO 2019/187933

(57) **Abstract**

A health assistance system 60 includes: an examination value prediction means 61 that predicts a future examination value for a prediction subject who is a designated person or a predetermined person using a prediction model specifying the relationship between a plurality of variables corresponding to one or more items relating to the current or past lifestyle habits of a certain person and a future examination value of the person; and a sheet output means 62 that generates and outputs, on the basis of the result of prediction, an information providing sheet for each subject being assisted who is considered the subject of assistance among prediction subjects, the information providing sheet including information for improving the lifestyle habits, preventing disorders, or promoting health of the subject being assisted.

## Description

### Technical Field

The present invention relates to a health assistance system, an information providing sheet output device used therefor, a health assistance method, and a health assistance program.

### Background Art

In recent years, with the declining birthrate and aging of the population, it is an important issue to extend healthy life expectancy while reducing medical expenses increasing nationwide.

For example, in Japan, a part of the guideline for health services was revised in March 2014. Based on this, it is desired to achieve effective and efficient health services in line with a plan-do-check-act (PDCA) cycle using health and medical information. In other words, it is required to enhance effectiveness of health services not by blindly implementing health services but by conducting a scientific approach using data.

However, it is said that it is not easy for an individual to raise interest in his/her own health and to review his/her lifestyle habits. For example, information that can be acquired by an individual regarding his/her health and lifestyle habits is limited. For example, it is difficult for an individual to specifically estimate necessity of review of his/her lifestyle habits and effects thereof and to specifically behave in order to prevent disorders and promote health on the basis of only results of annual medical checkups.

As technology relating to health prediction and health assistance, Non Patent Literature (NPL) 1 describes an example of a health risk prediction system that predicts a health risk on the basis of a result of a medical checkup and lifestyles. The health risk prediction system described in NPL 1 includes two subsystems, an examination value prediction system and an onset prediction system. The examination value prediction system indicates the degree of improvement in examination results associated with lifestyle improvement for a person having mild examination abnormalities and a lifestyle problem at the present time. In addition, the onset prediction system predicts a disease onset probability for a person not having examination abnormalities but having a biased lifestyle at the present time in each of a case where the person continues to have his/her undesirable lifestyle and a case when the person improves his/her lifestyle. According to NPL 1, health assistance by either of the subsystems is interactively performed between a medical staff and a medical examinee, and the medical examinee can recognize effects of his/her behavior change.

In addition, Patent Literature (PTL) 1 describes a method for assisting a target person in selecting a health program for health management, health promotion, prevention and treatment of a disease, and the like, the health program allowing the target person to continue participating in the program without feeling any burden. The method described in PTL 1 presents a program having contents close to behavior record of a target person from among a plurality of health programs by utilizing participation record information of a participant in a health program.

In addition, PTL 2 describes an example of a method for presenting a relationship between a biological index and diet or behavior for the purpose of giving a participant in guidance a confidence (self-efficacy) to perform lifestyle habit-improving behavior. In the method described in PTL 2, a rate of change of a biological index with respect to the intake amount of food and presence/absence of behavior is computed by multiple regression analysis using record data in which a value of a biological index and a record amount (the intake amount of food and the number of behaviors) satisfy predetermined conditions as a target. Then, on the basis of the computed rate of change, the food and behavior are ranked and output.

### Citation List

### Patent Literature

PTL 1: Japanese Patent Application Laid-Open No. 2015-103039
PTL 2: Japanese Patent Application Laid-Open No. 2013-190915

### Non Patent Literature

NPL 1: Hidetaka Takahashi, Katsumi Yoshida, "Health Risk Appraisal (HRA) for Lifestyle Improvement", Journal of Japan Society OF Ningen Dock (JHD) Vol. 11 No. 4 1997, p. 123-128.

### Summary of Invention

### Technical Problem

A problem is how to urge a person who is not highly interested in his/her own health, typified by a person who has not taken an examination itself for a long time, a person who takes an examination but does not participate in insurance guidance or an improvement program after the examination, and the like, to change his/her behavior. It is assumed that such a person may have little or no examination record of himself/herself, or little or no participation record of himself/herself in a subsequent improvement program. However, in order to improve effectiveness of health services in the whole of a nation or the whole of an organization, it is important to urge precisely such a person to change his/her behavior in order to prevent his/her own disorders and promote his/her health (for example, taking a medical checkup, participating in health guidance, and improving his/her lifestyle habits if necessary).

For example, the method described in NPL 1 is based on a premise that an examination value of a person can be acquired. Therefore, by the method described in NPL 1, it is not possible to urge a person who cannot acquire an examination value to change his/her behavior so as to take a medical checkup. That is, the method described in NPL 1 is merely to make a medical examinee aware of the current situation and to motivate the medical examinee to improve his lifestyle at a site of health guidance or the like after the person takes a medical checkup. For this reason, particularly in a case where it is tried to urge even a person having little or no examination record or little or no participation record in a subsequent improvement program because the person has low interest in his/her own health or does not have appropriate information regarding health services even if the person's interest is not low to change his/her behavior, the method described in NPL 1 is insufficient.

Similarly, the methods described in PTLs 1 and 2 also require a record value of behavior by a person himself/herself, and therefore are still insufficient in a case where it is tried to urge the above-described person to change his/her behavior.

In view of the above-described problems, an object of the present invention is to provide a health assistance system, an information providing sheet output device, a health assistance method, and a health assistance program, capable of appropriately assisting many people including a person having low interest in his/her own health or does not have appropriate information regarding health services even if the person's interest is not low in changing their behaviors in order to prevent their disorders and promote their health.

### Solution to Problem

A health assistance system according to the present invention is characterized by including: an examination value prediction means which predicts a future examination value for a prediction subject who is a designated person or a predetermined person using a prediction model specifying the relationship between a plurality of variables corresponding to one or more items relating to the current or past lifestyle habits of a certain person and a future examination value of the person; and a sheet output means which generates and outputs, on the basis of the result of prediction, an information providing sheet for each subject being assisted who is considered the subject of assistance among prediction subjects, the information providing sheet including information for improving the lifestyle habits, preventing disorders, or promoting health of the subject being assisted.

In addition, an information providing sheet output device according to the present invention is characterized by including: a storage means which stores prediction result data indicating a result of predicting a future examination value for a prediction subject who is a designated person or a predetermined person using a prediction model specifying the relationship between a plurality of variables corresponding to one or more items relating to the current or past lifestyle habits of a certain person and a future examination value of the person; and a sheet output means which generates and outputs, on the basis of the result of prediction, an information providing sheet for each subject being assisted who is considered the subject of assistance among prediction subjects, the information providing sheet including information for improving the lifestyle habits, preventing disorders, or promoting health of the subject being assisted.

In addition, a health assistance method according to the present invention is characterized by predicting a future examination value for a prediction subject who is a designated person or a predetermined person using a prediction model specifying the relationship between a plurality of variables corresponding to one or more items relating to the current or past lifestyle habits of a certain person and a future examination value of the person, and generating and outputting, on the basis of the result of prediction, an information providing sheet for each subject being assisted who is considered the subject of assistance among prediction subjects, the information providing sheet including information for improving the lifestyle habits, preventing disorders, or promoting health of the subject being assisted.

In addition, a health assistance program according to the present invention is characterized by causing a computer to execute: a process of predicting a future examination value for a prediction subject who is a designated person or a predetermined person using a prediction model specifying the relationship between a plurality of variables corresponding to one or more items relating to the current or past lifestyle habits of a certain person and a future examination value of the person; and a process of generating and outputting, on the basis of the result of prediction, an information providing sheet for each subject being assisted who is considered the subject of assistance among prediction subjects, the information providing sheet including information for improving the lifestyle habits, preventing disorders, or promoting health of the subject being assisted.

### Advantageous Effects of Invention

According to the present invention, it is possible to appropriately assist many people in changing their behaviors in order to prevent their disorders and promote their health.

### Brief Description of Drawings

[FIG. 1] FIG. 1 is a block diagram showing a configuration example of a health assistance system of a first exemplary embodiment.
[FIG. 2] FIG. 2 is a flowchart showing an example of operation of the health assistance system of the first exemplary embodiment.
[FIG. 3] FIG. 3 is a flowchart showing another operation example of the health assistance system of the first exemplary embodiment.
[FIG. 4] FIG. 4 is a block diagram showing another configuration example of the health assistance system of the first exemplary embodiment.
[FIG. 5] FIG. 5 is an explanatory diagram showing an example of a method for designating an output condition by a user.
[FIG. 6] FIG. 6 is a flowchart showing an example of a processing flow of a target selection process.
[FIG. 7] FIG. 7 is a flowchart showing an example of the processing flow of the target selection process.
[FIG. 8] FIG. 8 is an explanatory diagram showing an image of utilizing and applying an information providing sheet.
[FIG. 9] FIG. 9 is a schematic image of the information providing sheet.
[FIG. 10] FIG. 10 is an explanatory diagram showing a display example of a medical checkup result prediction of a certain examination item.
[FIG. 11] FIG. 11 is an explanatory diagram showing an example of information indicating a relationship between each examination value and a disease.
[FIG. 12] FIG. 12 is an explanatory diagram showing a display example of a change in a prediction value before and after a review as one kind of lifestyle improvement proposing information.
[FIG. 13] FIG. 13 is an explanatory diagram showing an example of a utilization scene of a series of processes from future prediction to sheet output.
[FIG. 14] FIG. 14 is a schematic block diagram showing a configuration example of a computer according to an exemplary embodiment of the present invention.
[FIG. 15] FIG. 15 is a block diagram showing an outline of a health assistance system of the present invention.
[FIG. 16] FIG. 16 is a block diagram showing an outline of an information providing sheet output device of the present invention.

### Description of Embodiments

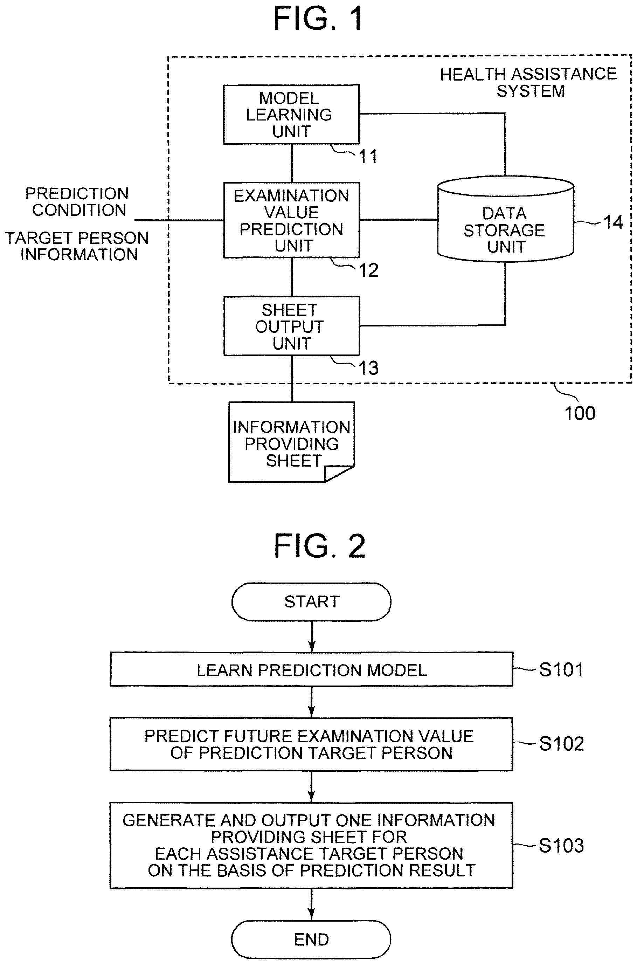

Hereinafter, an exemplary embodiment of the present invention will be described with reference to the drawings. FIG. 1 is a block diagram showing a configuration example of a health assistance system of the present exemplary embodiment. A health assistance system 100 shown in FIG. 1 includes a model learning unit 11, an examination value prediction unit 12, a sheet output unit 13, and a data storage unit 14.

The model learning unit 11 learns a prediction model for predicting a future examination value of a designated target person. Here, the prediction model is not particularly limited as long as being a model defining at least a relationship between a plurality of variables including one or more items relating to current or past lifestyle habits (hereinafter, referred to as lifestyle habit items) of a certain target person and a future examination value of the target person. The prediction model may be, for example, a model in which, for a predetermined examination item, one or more variables respectively corresponding to an examination value of the target person at a certain point in the past (for example, an examination value obtained in the latest medical checkup), and one or more inquiry items (lifestyle habit items) relating to lifestyle habits of the target person are at least used as explanatory variables, and a future examination value of the target person is predicted as an obj ective variable. Note that the explanatory variables are not limited to the above variables, and for example, may further include one or more variables indicating each examination value for a predetermined period in the past (or obtained by a predetermined number of past medical checkups) or a variable indicating a value of an item regarding attributes of the target person. Examples of the item regarding attributes of the target person include sex and age (or generation).

The model learning unit 11 may generate and learn the prediction model by performing big data analysis utilizing artificial intelligence (AI) technology, for example, on record data held by an organization to which an assistance target person belongs (local government, company, health insurance association, and the like) and including at least a medical checkup result and an inquiry result at the time of the medical checkup.

A learning method performed by the model learning unit 11 is not particularly limited, but various types such as supervised learning, unsupervised learning, semi-supervised learning, and reinforcement learning can be considered. In addition, as a machine learning algorithm, a neural network that is one type of general supervised learning may be utilized, or another algorithm (for example, support vector machine, deep learning, Gaussian process, decision tree, or random forest) may be utilized. In addition, examples of the prediction model include a linear model, a piecewise linear model, a regression model, a logistic regression model, a multiple regression model, and a multiple regression model with case classification.

The model learning unit 11 may learn the prediction model, for example, by utilizing a learning device that can specify the degree of contribution of each of a plurality of explanatory variables to an objective variable by machine learning using predetermined training data (record data). At this time, the model learning unit 11 may learn the prediction model using at least one of sparsity and regularization as a constraint. As an example, the model learning unit 11 may learn the prediction model by utilizing one of learning devices based on multiple regression analysis with case classification, the learning device performing heterogeneous mixture learning that solves a nonlinear and sparse problem with high accuracy in a white box. In addition, the model learning unit 11 may specify, as a result of learning, the magnitude (absolute value) of a coefficient of each variable of a prediction formula of the prediction model as the degree of contribution. At this time, a variable in which the degree of contribution is zero may be excluded from the explanatory variables.

Information (various parameters) of the learned prediction model obtained by the model learning unit 11 is stored in, for example, the data storage unit 14. Note that the number of the prediction models learned by the model learning unit 11 is not limited to one. That is, in a case where there is a plurality of examination values to be predicted, the model learning unit 11 learns the prediction model for each of examination items.

The examination value prediction unit 12 predicts a future examination value of a designated prediction target person or a predetermined prediction target person using the learned prediction model obtained by the model learning unit 11. At this time, the examination value prediction unit 12 may receive target person information which is information for specifying a value of each explanatory variable to be input to the prediction target person and further to the prediction model and a prediction condition, and may make a prediction on the basis of the received information. Note that there may be a plurality of prediction target persons. At this time, it is also possible to designate conditions of the prediction target person and a corresponding range, such as all the persons belonging to a certain organization, in advance. Note that a user of the present system can also designate conditions of the prediction target person and a corresponding range each time.

The target person information may be, for example, an individual identifier (ID) or a user ID in the organization, capable of specifying attribute data indicating attributes of a prediction target person and record data. In addition, it is also possible to directly designate a value of each explanatory variable to be input to the prediction model as the target person information. Examples of the prediction condition include designation regarding a prediction time point at which an examination value is predicted (designation of a time point at which an examination value is predicted, designation of each prediction time point in a case where there is a plurality of prediction time points, and the like). In addition, other examples of the prediction condition include designation of whether or not to utilize record data regarding lifestyle habits, and designation of a value to be used for each lifestyle habit item in a case where the record data is not utilized. Note that the prediction condition may be a combination of these examples.

Note that for example, in a case where there is no record data of the prediction target person, even if the target person information is received, a value of an explanatory variable corresponding to the prediction target person cannot be necessarily specified for all the explanatory variables. Therefore, in a case where there is an explanatory variable for which a value corresponding to the prediction target person cannot be specified, the examination value prediction unit 12 may search for another person who is similar to the prediction target person on the basis of specified information, and may utilize a value of the another person who has been searched for. At this time, the examination value prediction unit 12 preferably searches for another person having similarity in any one of attributes, tendency of past examination values, and lifestyle habits, or a combination thereof. Note that a search target is not particularly limited. For example, the search target may be external data such as data on the Internet, or may be data held in a database in an organization to which a target person assistance person belongs.

A prediction result by the examination value prediction unit 12 is stored in the data storage unit 14 in association with information of the prediction target person together with the value of the explanatory variable used for the prediction and the prediction condition. Note that in a case where there is a plurality of examination values to be predicted, the examination value prediction unit 12 predicts a future examination value for each examination item using a prediction model corresponding to the examination item. Note that an example of the prediction by the examination value prediction unit 12 (who is targeted, at what timing, what is predicted, and the like) will be described later.

The sheet output unit 13 generates and outputs one information providing sheet for each assistance target person on the basis of the prediction result by the examination value prediction unit 12. Here, the information providing sheet is a sheet summarizing information for improving lifestyle habits of the assistance target person, preventing his/her disorder, or promoting his/her health, the information being specified on the basis of the prediction result and directed to each assistance target person. Note that an example of the information providing sheet output by the sheet output unit 13 (specific contents of the information providing sheet, output format, and the like) will be described later.

In addition, the prediction result by the examination value prediction unit 12 can be used for model verification by the model learning unit 11. For example, the model learning unit 11 may divide a predetermined number of pieces of past record data into learning data and evaluation data, may learn a prediction model by utilizing the learning data, may perform model evaluation by comparing a prediction value obtained from the learned prediction model by utilizing the evaluation data with a record value, and may perform re-learning as necessary. At this time, an examination value obtained after obtaining a prediction value can be included as the record value to be compared.

Next, operation of the present exemplary embodiment will be described. FIG. 2 is a flowchart showing an example of operation of the health assistance system of the present exemplary embodiment. In the example shown in FIG. 2, first, the model learning unit 11 learns a prediction model for each examination value to be predicted (step S101). In this example, information of the learned prediction model is stored in the data storage unit 14.

Subsequently, the examination value prediction unit 12 receives target person information and a prediction condition, and predicts a future examination value of a prediction target person using a prediction model on the basis of the received information (step S102). In this example, the prediction result by the examination value prediction unit 12 is stored in the data storage unit 14 in association with information of the prediction target person together with a value of an explanatory variable used for the prediction and the prediction condition.

Finally, the sheet output unit 13 generates and outputs one information providing sheet for each assistance target person on the basis of the prediction result by the examination value prediction unit 12 (step S103).

Note that in the above description, a model learning phase (step S 101), a prediction phase (step S102), and a sheet generating phase (step S103) are described as a series of operations, but these phases can also be operated independently. In this case, the model learning phase only needs to be performed at least once before the prediction phase. In addition, the prediction phase only needs to be performed at least once before the sheet generating phase. In addition, exchange of information between the phases only needs to be performed, for example, via the data storage unit 14.

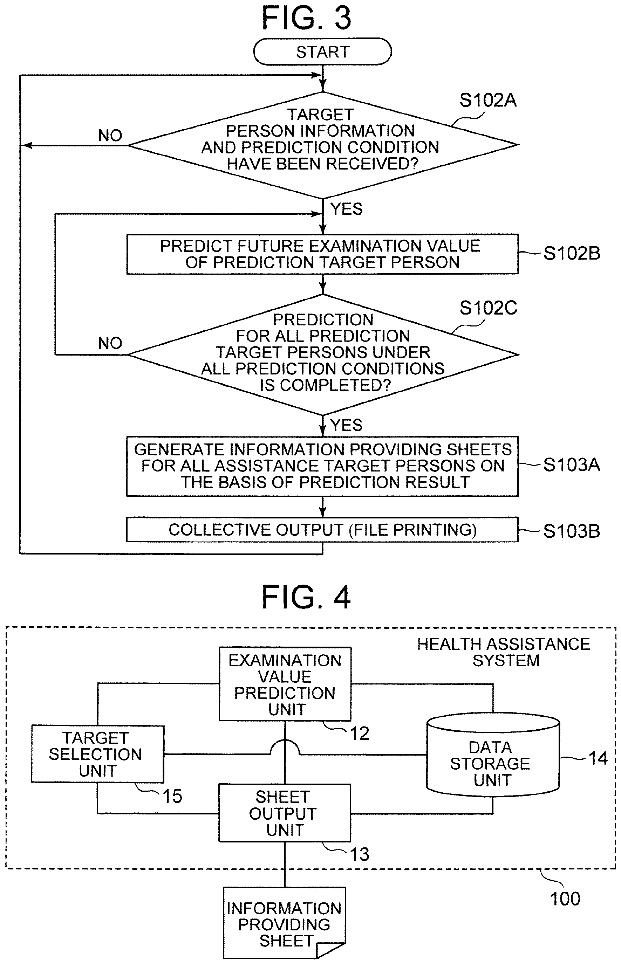

In addition, FIG. 3 is a flowchart showing another operation example of the health assistance system of the present exemplary embodiment. Note that in the example shown in FIG. 3, it is assumed that a learned prediction model is stored in the data storage unit 14, and the model learning phase (step S101) is omitted.

In this example, if target person information and a prediction condition are received (Yes in step S102A), the examination value prediction unit 12 repeats a process of predicting a future examination value of a prediction target person (step S102B) until predictions for all the prediction target persons under all the prediction conditions are completed (No in step S102C).

If the predictions for all the prediction target persons under all the prediction conditions are completed (Yes in step S102C), the sheet output unit 13 generates information providing sheets for all the assistance target persons on the basis of a prediction result by the examination value prediction unit 12 (step S103A). At this time, the examination value prediction unit 12 may generate information providing sheets for a plurality of assistance target persons collectively in one file.

When the information providing sheets for all the assistant persons are generated, the information providing sheets are collectively output (for example, file printing) (step S103B).

In addition, FIG. 4 is a block diagram showing another configuration example of the health assistance system of the present exemplary embodiment. As shown in FIG. 4, the health assistance system 100 may further include a target selection unit 15 in the configuration shown in FIG. 1. Note that in the example shown in FIG. 4, the model learning unit 11 is omitted because information of a prediction model learned for each examination item is stored in the data storage unit 14 in advance. However, the model learning unit 11 can be included similarly to the first exemplary embodiment.

The target selection unit 15 selects (specifies) an assistance target person for whom an information providing sheet is generated in the sheet output unit 13 or an assistance target item (for example, an examination item and a lifestyle habit item that urges a person to make a change in order to improve the examination item). As a simulation, the target selection unit 15 causes the examination value prediction unit 12 to make a prediction on a predetermined assistance target item candidate while changing a prediction target person or a prediction condition, and on the basis of a result of the prediction, selects a prediction target person or an assistance target item. The prediction target person and the prediction condition to be simulated can be set in advance or can be provided by a user. In addition, the target selection unit 15 can also receive designation of an assistance target item candidate and an output condition (for example, a condition for selecting an assistance target person or an assistance target item) from a user.

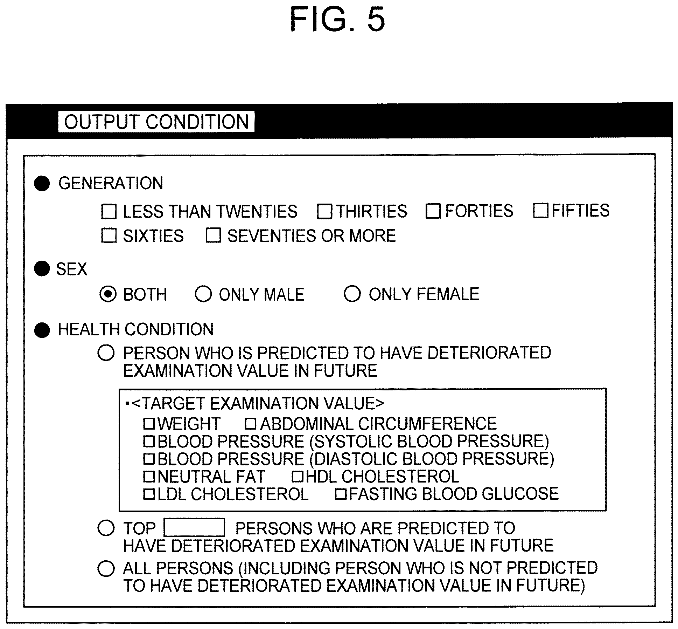

FIG. 5 is an explanatory diagram showing an example of a method for designating an output condition by a user. As shown in FIG. 5, a screen for a user to designate an output condition may be prepared in advance.

The examination value prediction unit 12 or the sheet output unit 13 can also perform a part or the whole of the selection process in the target selection unit 15.

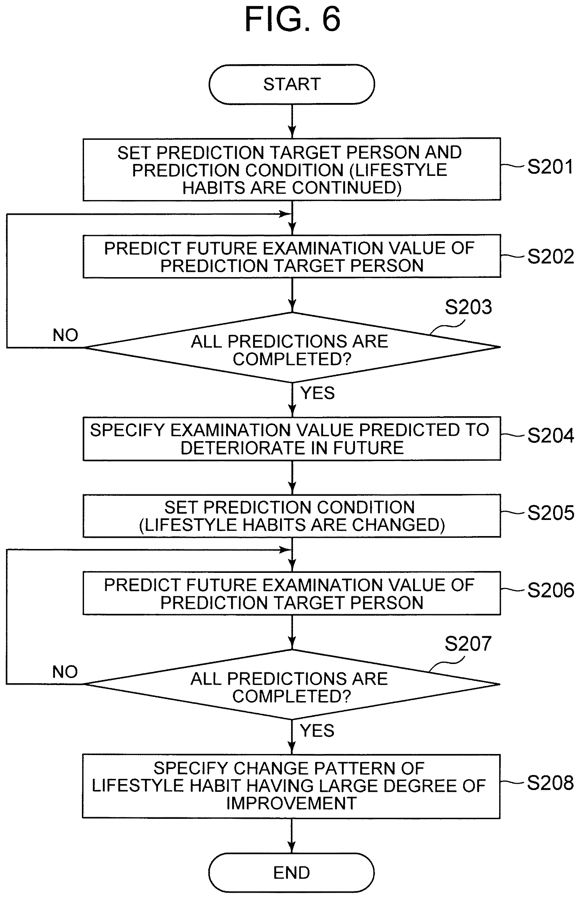

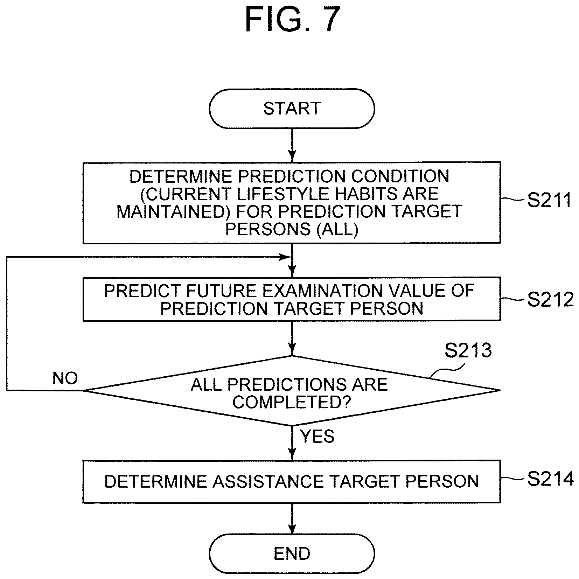

FIGs. 6 and 7 are flowcharts each showing an example of a processing flow of a target selection process. The example shown in FIG. 6 is an example of a process for selecting an assistance target item, and the example shown in FIG. 7 is an example of a process for selecting an assistance target person.

In the example shown in FIG. 6, first, the target selection unit 15 sets a prediction condition for a designated prediction target person or a predetermined prediction target person such that an examination value when the current (or latest) lifestyle habits are maintained is predicted for all the examination items (step S201).

As a result of step S201, target person information indicating a prediction target person and a prediction condition are output to the examination value prediction unit 12. Hereinafter, the prediction condition set in step S201 may be referred to as a first prediction condition.

When receiving the target person information and the prediction condition (first prediction condition), the examination value prediction unit 12 predicts a future examination value of a prediction target person using a prediction model on the basis of the received information (step S202). The prediction process itself of the examination value prediction unit 12 is similar to that in step S102 except that a value of an explanatory variable input to the prediction model changes depending on the target person information and the prediction condition.

In a succeeding step S203, it is determined whether or not all the predictions indicated by the target person information and the prediction condition have been completed. If all the predictions have been completed (Yes in step S203), the process proceeds to step S204. If all the predictions have not been completed (No in step S203), the process returns to step S202, and a prediction is repeated. The determination of completion of the prediction may be performed by the examination value prediction unit 12 or can be performed by the target selection unit 15 that has received a prediction value as a prediction result.

In step S204, the target selection unit 15 determines, for each prediction target person, an examination value predicted to deteriorate in the future as an examination item to be an assistance target item on the basis of a prediction result based on the first prediction condition.

The specification of the examination value predicted to deteriorate in the future may be performed, for example, on the basis of the degree of change of an examination value determined by comparing the latest examination value used for the prediction with the examination value predicted in step S202 for each of the predicted examination items. For example, the examination value is specified by selecting an examination item in which the determined degree of change is equal to or larger than a predetermined threshold or by selecting a predetermined number of examination items in descending order of the degree of change. Here, the degree of change may be a change amount of a predicted examination value with respect to the latest examination value in a deteriorating direction, or a rate of change that is a ratio of the change amount in a value range that the examination item can take. In addition, as another specification method, a reference value (an upper limit reference value, a lower limit reference value, or the like) that is determined to have deteriorated may be set in advance for each examination item, and an examination item in which a predicted examination value is in a deteriorated state (an examination item in which an examination value exceeds the upper limit reference value or an examination item in which an examination value exceeds the lower limit reference value) may be selected from among the predicted examination items, or a predetermined number of examination items may be further selected from among the selected examination items in descending order of the degree of change. At this time, it is also possible to select an examination item in which the latest examination value is not in a deteriorated state but the predicted examination value is in a deteriorated state.

When an examination item to be an assistance target item is determined for each prediction target person, the target selection unit 15 sets a prediction condition again for each prediction target person such that an examination value for each change pattern is predicted for each examination item to be an assistance target item in a case where lifestyle habits are changed (step S205). In step S205, a prediction condition is set such that all possible change patterns for combinations of a plurality of variables corresponding to lifestyle habit items are prediction targets.

As a result of step S205, target person information indicating a prediction target person and a prediction condition are output to the examination value prediction unit 12. Hereinafter, the prediction condition set in step S205 may be referred to as a second prediction condition.

When receiving the target person information and the prediction condition (second prediction condition), the examination value prediction unit 12 predicts a future examination value of a prediction target person using a prediction model on the basis of the received information (step S206). The prediction process itself of the examination value prediction unit 12 is similar to that in step S102 except that a value of an explanatory variable input to the prediction model changes depending on the target person information and the prediction condition.

In a succeeding step S207, it is determined whether or not all the predictions indicated by the prediction information and the prediction condition have been completed. If all the predictions have been completed (Yes in step S207), the process proceeds to step S208. If all the predictions have not been completed (No in step S207), the process returns to step S206, and a prediction is repeated. Similarly to step S203, the determination of completion of the prediction may be performed by the examination value prediction unit 12 or can be performed by the target selection unit 15 that has received a prediction value as a prediction result.

In step S208, the target selection unit 15 determines, on the basis of a prediction result based on the second prediction condition, a lifestyle habit item changed in a change pattern of a lifestyle habit having a large degree of improvement as a target of change assistance in the assistance target item for each examination item to be an assistance target item of a prediction target person.

The lifestyle habit item may be specified, for example, for each of the predicted examination items, on the basis of the degree of improvement for each change pattern, determined by comparing an examination value predicted in step S202 (a prediction value when current lifestyle habits are maintained) with a prediction value in each change pattern of the lifestyle habits. For example, the lifestyle habit item can be specified by selecting a lifestyle habit change pattern in which the degree of improvement is equal to or larger than a predetermined threshold or by selecting a predetermined number of change patterns in descending order of the degree of improvement. Here, the degree of improvement may be a change amount of a prediction value (a prediction value in each change pattern) when at least some of the lifestyle habits are changed with respect to a prediction value when the current lifestyle habits are maintained in an improving direction, or a rate of change that is a ratio of the change amount in a value range that the examination item can take.

In addition, in the example shown in FIG. 7, first, the target selection unit 15 sets a prediction condition for a designated examination item or a predetermined examination item such that an examination value when the current (or latest) lifestyle habits are maintained is predicted for prediction target persons who are all the designated target person candidates or all the predetermined target person candidates (step S211). Here, the designated target person candidates or the predetermined target person candidates may be a set of arbitrary candidates designated as a target person candidate set by a user, or may be a set of arbitrary candidates determined in advance as a target person candidate set (for example, a set of persons belonging to an organization that is a user of the system). In addition, the designated target person candidates or the predetermined target person candidates may be a set of candidates including those who meet an extraction condition designated by a user, selected from these sets of arbitrary candidates.

As a result of step S211, target person information indicating a prediction target person and a prediction condition are output to the examination value prediction unit 12. Hereinafter, the prediction target person set in step S211 may be referred to as a first prediction target person.

When receiving the target person information (information indicating the first prediction target person) and the prediction condition, the examination value prediction unit 12 predicts a future examination value of a prediction target person using a prediction model on the basis of the received information (step S212).

In a succeeding step S213, it is determined whether or not all the predictions indicated by the target person information and the prediction condition have been completed. If all the predictions have been completed (Yes in step S213), the process proceeds to step S214. If all the predictions have not been completed (No in step S213), the process returns to step S212, and a prediction is repeated.

In step S214, the target selection unit 15 determines an assistance target person on the basis of a prediction result for each of the first prediction target persons.

The target selection unit 15 may determine a top predetermined number of persons or a person satisfying a predetermined condition as an assistance target person, for example, after rearranging the first prediction target persons in order in which a prediction value is becoming better (for example, in order in which a change width is decreasing or a difference from a standard value is decreasing). At this time, the target selection unit 15 can also perform only rearrangement and determine all the first prediction target persons as assistance target persons.

At the time of rearrangement, in a case where there are prediction values for a plurality of examination items, the target selection unit 15 preferably comprehensively determines prediction results thereof and rearranges the first prediction target persons in order in which a prediction value is becoming better. In addition, examples of the predetermined condition include a person having an examination value exceeding the reference value in a deteriorated state, and a person who shifts from a state that is not a deteriorated state to a deteriorated state.

The target selection unit 15 may select an assistance target person or an assistance target item (an examination item and a lifestyle habit item to be a target of change assistance for improving the examination item), for example, by such a process.

Next, some examples of a prediction by the examination value prediction unit 12 and some examples of sheet output by the sheet output unit 13 will be described. Note that in a part of the following description, an example will be described in which the examination value prediction unit 12 determines a prediction target person and the sheet output unit 13 determines an assistance target person and an assistance target item. However, in a configuration including the target selection unit 15, the target selection unit 15 can also make these determinations.

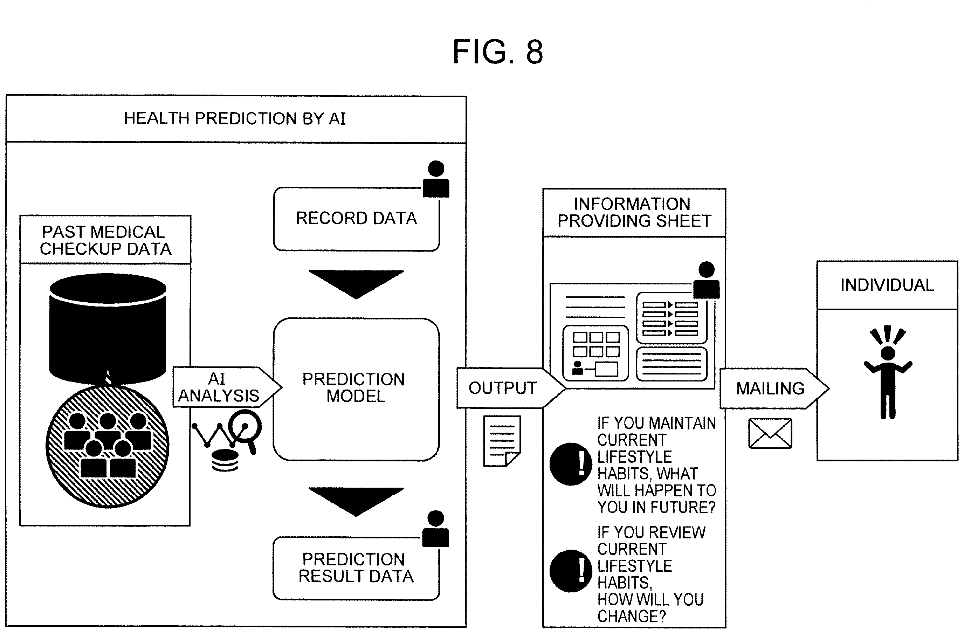

FIG. 8 is an explanatory diagram showing an image of utilizing and applying an information providing sheet. As shown in FIG. 8, the health assistance system 100 of the present exemplary embodiment predicts a future health condition of an individual using a prediction model (a prediction model corresponding to each examination item) derived from past accumulated record data of medical checkups, and generates prediction result data. Then, the information providing sheet indicating the prediction result, a lifestyle habit improvement item derived from the prediction result, and the like is provided to the individual. The information providing sheet is, for example, printed and mailed to the individual. By presenting, with such an information providing sheet, a specific future image such as a predicted future when the current lifestyle habits are continued, a specific improvement item for improving the current lifestyle habits, and effects thereof by prediction, a person is a motivated to improve his/her lifestyle or to participate in health services.

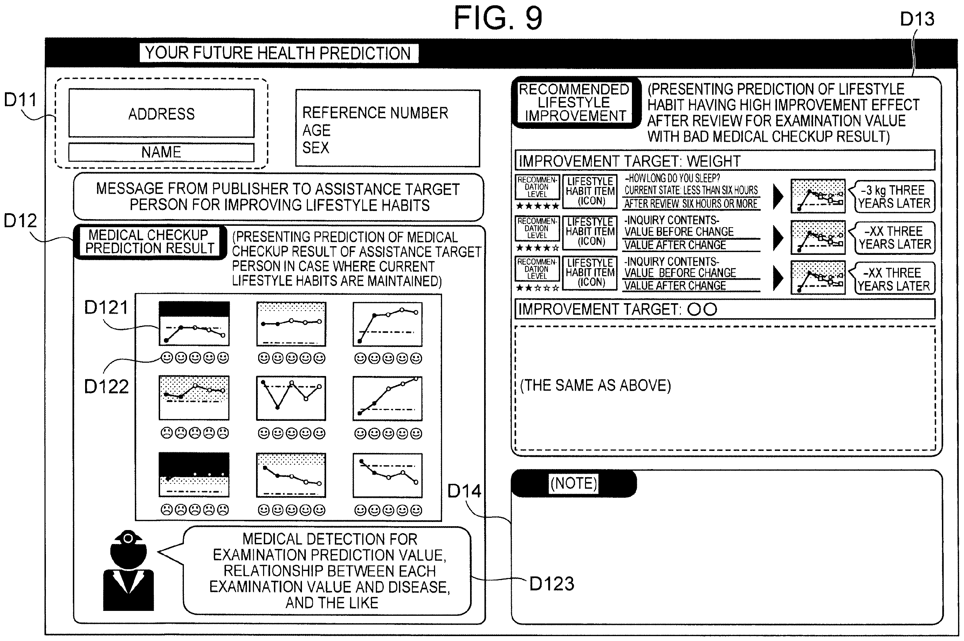

In addition, FIG. 9 is a schematic image of the information providing sheet. As shown in FIG. 9, the information providing sheet may include mailing destination information D11 for mailing. The mailing destination information D11 includes, for example, an address and a name of an assistance target person, and is arranged at a position matched with a window frame of an envelope with a window.

In addition, the information providing sheet may include a medical checkup result prediction D12 indicating a prediction of a health checkup result of an assistance target person in a case where the current lifestyle habits are maintained. The medical checkup result prediction D12 may include a graph D121 showing a transition of examination values including prediction values at a plurality of future time points such as one year ahead and two years ahead for each examination item examined in a medical checkup. Note that the graph D121 may display a face mark D122 in association with each time when a prediction value or a record value is obtained and corresponding to a health level at the time. The health level of each examination value may be classified, for example, into three levels (normal area/health guidance area satisfying health guidance determination value/consultation recommendation area satisfying consultation recommendation determination value) based on the "Medical Checkup Determination Value of Medical Checkup Examination Item" of the Ministry of Health, Labor, and Welfare. Furthermore, in addition to displaying the face mark D122, for example, a graph background may be displayed in different colors on the basis of the determination value (see, for example, shaded display in the graph in the figure).

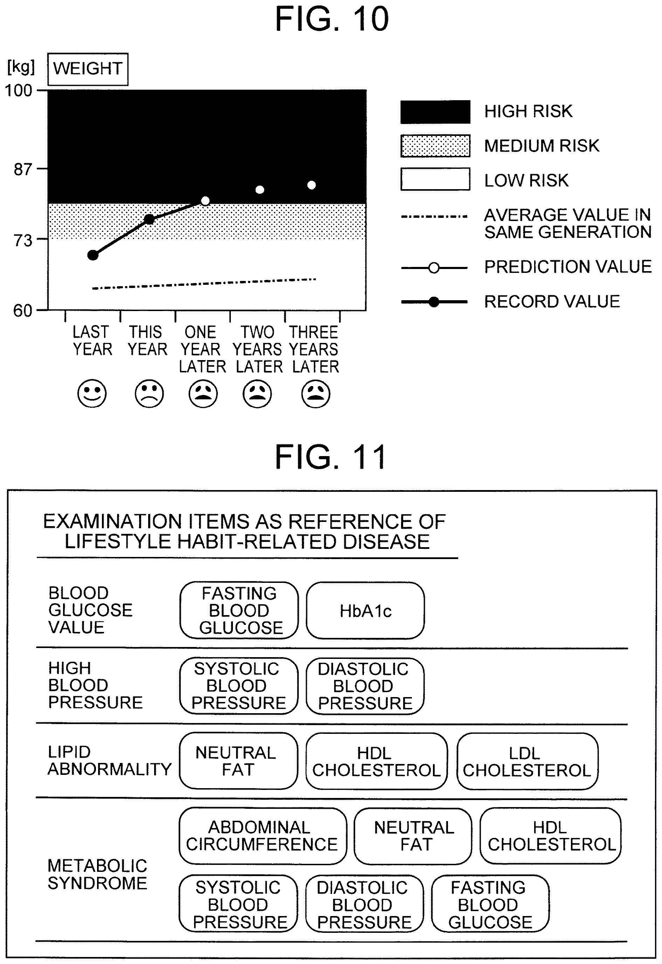

In the medical checkup result prediction D12, for example, in order to intuitively imagine what kind of health condition a person himself/herself will have in the future, medical checkup results (record values) of last year and this year, and an examination value (prediction value) of each year one to n years later may be displayed continuously in a graph for each examination item. Note that n = 3 is satisfied in this example. FIG. 10 shows a display example of a medical checkup result prediction of a certain examination item in an information providing sheet. As shown in FIG. 10, in the medical checkup result prediction D12, not only a transition of a record value and a prediction value of an assistance target person is displayed in a graph, but also an average value and the like of people in the same generation in a target organization and the like may also be displayed in a graph.

In addition, in the medical checkup result prediction D12, information D123 indicating a medical viewpoint for an examination value including a prediction value and a transition of the examination value, a relationship between each examination value and a disease, and the like may be presented. The information D123 may include, for example, for an examination value predicted to deteriorate, a message explaining risks of a lifestyle habit-related disease and a disease associated with deterioration of the examination value, a list of specific disease risk names, and the like. FIG. 11 shows an example of the information D123 indicating a relationship between each examination value and a disease. By including such information, it is possible for an assistance target person to find what kind of disease is caused by deterioration of each examination value.

In addition, the information providing sheet may include lifestyle improvement proposing information D13 indicating a lifestyle habit item specified as a result of a simulation for an examination value with a large deviation from a reference value and having a high improvement ratio after review of the examination value, and contents thereof together with an improvement effect. Note that in the example shown in FIG. 9, a simulation is performed on two examination values, a first examination value having the largest deviation from the reference value and a second examination value having the second largest deviation from the reference value, and the lifestyle improvement proposing information D13 is presented.

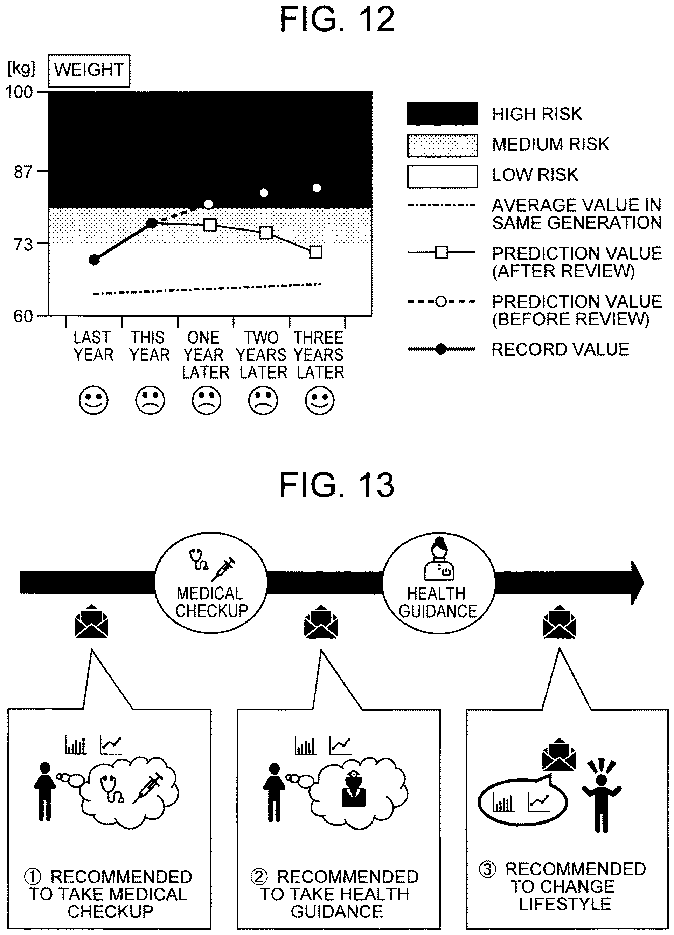

The lifestyle improvement proposing information D13 provides a display, for example, such that it is possible to specifically find how a prediction value changes when lifestyle habits (exercise, diet, drinking, sleep, and the like) are reviewed. For example, in the lifestyle improvement proposing information D13, for each of the first examination value and the second examination value, a top predetermined number of lifestyle habit items having a large degree of improvement after the review with respect to the examination value are specified, and then for each of the lifestyle habit items, current contents (answer to an inquiry), contents after the review, a graph showing a change in a prediction value before and after the review, and a recommendation level may be displayed. FIG. 12 shows a display example of a change in a prediction value before and after the review as one kind of the lifestyle improvement proposing information D13. In the example shown in FIG. 12, prediction values before and after the review are displayed in one graph such that an assistance target person can compare and confirm how an examination value changes after the review as compared with an examination value before the review. In this way, by presenting the examination value after the review as well, it is possible to indicate an effect in a case of continuing to improve lifestyle habits.

At this time, lifestyle habit items to be presented may be displayed in a ranking form in descending order of a recommendation level, in which the degree of the degree of improvement is scored. Such a display can be used as a reference for generating a plan for improving lifestyles.

Furthermore, in addition to those described above, the information providing sheet may also include a remarks column D14 in which tips for health promotion (for example, introduction of activities in a target organization), contact information, and the like are presented.

The information providing sheet is generated after the size of the information providing sheet is adjusted such that one sheet is output for each assistance target person when the information is output. The information providing sheet may have, for example, A3 size when the information providing sheet is opened (A4 size when the information providing sheet is folded).

According to such an information providing sheet, it is possible to provide a prediction value based on record data and big data, a change in a prediction value before and after the improvement, and the like. Therefore, a target assistance person can objectively grasp his/her own condition. For example, an assistance target person can grasp a health problem including a future situation, and a sense of crisis can be thereby produced. Furthermore, efficiency can be improved by presenting effective and specific activities (specific contents of review of lifestyle habits for improving an examination item) specified by AI. Furthermore, by also presenting a future prediction when the lifestyle habits are reviewed, an assistance target person can grasp a future image after his or her own activities, and therefore a sense of expectation can be produced. Through the production of a sense of crisis, the improvement of efficiency, and the production of expectation as described above, a trigger for changing a behavior is provided, and a person is encouraged to start a behavior (behavior change is accelerated).

In addition, FIG. 13 is an explanatory diagram showing an example of a utilization scene of a series of processes from future prediction to sheet output. As shown in FIG. 13, the processes may be performed before or during a period when a medical checkup is held by using a person who has not yet taken a medical checkup as a prediction target person (candidate for an assistance target person) among medical checkup target persons. By providing information to a person who has not yet taken a medical checkup during a period when a medical checkup is held, it is recommended to take a medical checkup, and an effect of improving a medical checkup ratio is expected. In addition, as shown in FIG. 13, the processes may be performed during a health guidance calling time by using a target person of the health guidance calling as a prediction target person (candidate for an assistance target person). By providing information during the health guidance calling time, it is recommended to take health guidance, and an effect of improving a medical checkup ratio is expected. In addition, as shown in FIG. 13, it is also possible to provide information at any timing after a medical checkup or health guidance is performed. For example, by providing information at a timing other than a period when a medical checkup or health guidance is performed, it is recommended to improve lifestyles, for example, motivation to continue guidance contents is improved, or a trigger for reviewing lifestyles of a person himself/herself by a means other than a medical checkup and health guidance is provided, and health promotion can be promoted.

When information is provided during a period when a medical checkup is held, for example, in a case where a medical checkup target person has taken a medical checkup in the latest past few years (past one to two years), a prediction may be made on the basis of the past medical checkup results of the medical checkup target person, and obtained prediction information (including a prediction result by a simulation and an analysis result of the prediction result) may be output as an information providing sheet. Meanwhile, in a case where the medical checkup target person has not taken a medical checkup in the last few years or has taken an irregular medical checkup even if the medical checkup target person has taken a medical checkup, it is conceivable that there is a deficiency in the past medical checkup results necessary for a prediction for the target person. In this case, the deficient part may be complemented with an average value of persons with the same age and same sex, then a prediction may be made, and the obtained prediction information may be output as an information providing sheet. In addition, the information providing sheet output at the timing may be sent to the target person while being enclosed with a medical checkup voucher (ticket indicating a medical checkup qualification). At this time, in order to facilitate enclosing, output control may be performed such that a sheet of each assistance target person is printed together with a medical checkup voucher of the assistance target person.

Even in a case where a person has a high medical checkup ratio, the person may have a predicted examination value in a deteriorated state although having the latest examination value not in a deteriorated state. Therefore, by providing information using future prediction to such a person, a disorder prevention effect can be expected. In addition, for a person having a low past medical checkup ratio, by providing information using future prediction to the person to increase interest in his/her own health, an effect of improving a medical checkup ratio can be expected.

In addition, when information is provided during a period when health guidance is held, for example, for a guidance target person (for example, a person with a finding for whom a medical checkup result of this time has been determined to include findings), a prediction may be made on the basis of a past medical checkup result of the target person, and the obtained prediction information may be output as an information providing sheet. At this time, in a case where there is a deficiency in the past medical checkup result, it is only required to make a prediction after complementing the deficient part with an average value of persons with the same age and same sex in a similar manner to the above. Furthermore, in addition to targeting all the guidance target persons, it is also possible to target a guidance target person who is likely to have a deteriorated examination values in the future. In addition, the information providing sheet output at the timing may be sent to the target person while being enclosed with a notification of calling for health guidance. At this time, in order to facilitate enclosing, output control may be performed such that a sheet of each assistance target person is printed together with the notification of calling to the assistance target person. In addition, the contents of the information providing sheet output at this time is preferably provided also to an instructor who provides health guidance, for example, by outputting the contents of the information providing sheet on a screen at the time of guidance.

By providing information using future prediction and deepening understanding of situations of a health guidance target person himself/herself at the time of calling to the person after the person takes a medical checkup, an increase in a participation ratio in health guidance can be expected.

In addition, when information is provided at any timing, for example, for a high-risk person in the future (a person who is predicted to be in a deteriorated state in the future even if the person is not currently in a deteriorated state), a prediction may be made on the basis of a past medical checkup result of the target person, and the obtained prediction information may be output as an information providing sheet. At this time, in a case where there is a deficiency in the past medical checkup result, it is only required to make a prediction after complementing the deficient part with an average value of persons with the same age and same sex in a similar manner to the above. Note that it is also possible to target a person who has all the past medical checkup results or a person who has registered at least the latest medical checkup result.

For example, a person who has a medical checkup result of this time is equal to or less than a reference value and is not a target of health guidance but is likely to have a medical checkup result exceeding the reference value in the near future may be extracted on the basis of the prediction result, and information may be provided to the person. As a result, an assistance target person can grasp his/her own health problem early without waiting for a next medical checkup and the like, which can be a trigger for reviewing lifestyle habits, and a health promotion effect can be expected.

The following are other examples of utilizing and applying the health assistance system of the present exemplary embodiment.

### •(Utilization and Application Example 1)

A prediction model obtained by analyzing big data with AI on the basis of medical checkup data indicating a medical checkup result of each individual held by an organization that provides health assistance to individuals (called a management organization), in which a result of predicting the future of an individual is output as one sheet utilizing a prediction model that predicts a future examination value using a past examination value, one or more lifestyle habit items, sex, and generation as explanatory variables, and provided to the individual.

### •(Utilization and Application Example 2)

Using explanatory variables (examination value, lifestyle habit item, sex, and age) obtained from medical checkup results for the latest two years of an individual as input, information obtained as a result of making a prediction up to three years in the future is output as a sheet.

At this time, a prediction may be made in a case where current lifestyle habits are continued. In addition, as a result of the prediction, at least a person who is predicted to have a bad future examination value may be an output target. In addition, the sheet may include information indicating the contents of review of lifestyle improvement and an effect thereof in association with each other, for example, with respect to an examination value predicted to deteriorate in the future, by what kind of lifestyle improvement, how much improvement effect can be obtained.

### •(Utilization and Application Example 3)

In addition, the sheet may include, as tips for health promotion, information indicating a specific means for improving lifestyle habits promoted by a management organization (for example, remarks column D14). Not only by presenting the contents of review of general lifestyle improvement, such as recommending exercise and diet review, but also by specifically presenting a means existing nearby, it is possible to increase a ratio of starting lifestyle improvement.

### •(Utilization and Application Example 4)

In addition, the sheet may include information indicating the contents of review of lifestyle improvement predicted to have a high improvement effect by extracting up to n (for example, 2) examination values predicted to deteriorate in the future (for example, lifestyle improvement proposing information D13). The n examination values to be extracted may be obtained by comparing the current examination value with an examination value at a future time point (not limited to one and the latest time point. For example, the time point may be three years later), and selecting n examination values in descending order of a deterioration ratio. By performing evaluation using the ratio, it is possible to equally evaluate examination values with different units and value ranges.

In addition, the examination values to be extracted at this time may be nine examination values, that is, weight, abdominal circumference, low-density lipoprotein (LDL) cholesterol, high-density lipoprotein (HDL) cholesterol, neutral fat, minimal blood pressure (diastolic blood pressure), maximal blood pressure (systolic blood pressure), Hemoglobin A1c (HbA1c), and fasting blood glucose.

### •(Utilization and Application Example 5)

In addition, when contents of review of lifestyle improvement predicted to have a high improvement effect are indicated for a certain examination value, all review patterns that can be taken as a combination of items relating to lifestyle improvement may be predicted, and the contents of review may be presented in descending order of a computed improvement effect (for example, in descending order of a computed degree of improvement) together with the degree of improvement.

### •(Utilization and Application Example 6)

In addition, the sheet may be generated and output on the basis of a result of the prediction made for a medical checkup target person of a predetermined medical checkup such as a regular medical checkup for a national health insurance member. In addition, the sheet may be generated and output for a person who has not yet taken a medical checkup or a person who irregularly takes a medical checkup in a predetermined number of past medical checkups. In addition, the sheet may have a column in which an address and a name are written for sending, or a column in which the identification number of a medical checkup target person is described, and may be sent together with a medical checkup ticket.

### •(Utilization and Application Example 7)

In addition, the sheet may be generated and output for a health guidance target person who has been determined to have findings after taking a predetermined medical checkup. In addition, the sheet may have a column in which an address and a name are written for sending, or a column in which the identification number of a medical checkup target person is described, and may be sent together with a notification of calling for health guidance.

### •(Utilization and Application Example 8)

In addition, the sheet may be generated and output for a person who has taken a predetermined medical checkup and has no problem with an examination value at the present time, but is predicted to have a deteriorated examination value in the future (for example, to have findings in the future).

In a case of a person who has taken a medical checkup and has no problem with an examination value at the present time, even if the person has bad lifestyle habits and is highly likely to have findings in the future, there is no trigger for approaching the person concerned in many cases. However, it is possible to send the sheet also to such a person. This makes it possible to raise awareness of the person concerned and to alert the person.

### •(Utilization and Application Example 9)

In addition, the assistance target person who is a target person for the sheet output may be limited to a person who is predicted to have a deteriorated examination value in the future. For example, current examination values and future examination values may be evaluated for all the candidates, and a predetermined number of top persons in descending order of an increasing ratio in a deteriorating direction (deterioration ratio) may be used as target persons. Note that such a limitation can be achieved, for example, by receiving setting of an output condition from a user (user in a management organization).

### •(Utilization and Application Example 10)

In addition, the assistance target person who is a target person of the sheet output may be narrowed down by a predetermined condition such as generation, sex, or an examination value that deteriorates. As a result, flexible assistance corresponding to health issues in a management organization, such as "a desire to focus on lifestyle improvement of young people in their twenties and thirties" or "a desire to focus on a blood glucose-related examination value in order to reduce incidence of diabetic patients", can be provided.

### •(Utilization and Application Example 11)

In addition, in order to be able to output a large number of sheets even if the number of assistance target persons is large, such as thousands or tens of thousands, output may be performed in a file including a plurality of pages in which one sheet is arranged on one page such that information of each assistance target person is included in one sheet. At this time, the maximum number of pages that can be included in one file may be set by setting a condition and the like. In a case where one file includes pages exceeding the maximum number of pages, it is also possible to divide the file automatically (a separate file can be generated).

As described above, according to the present exemplary embodiment, it is possible to increase interest in a person's own health, to produce a sense of crisis, and to collectively output prediction information that produces expectation by an improvement effect in one sheet in a compact manner. For this reason, it is possible to appropriately assist many people including a person has low interest in his/her own health or does not have appropriate information regarding health services even if the person's interest is not low in changing their behaviors in order to prevent their disorders and promote their health.

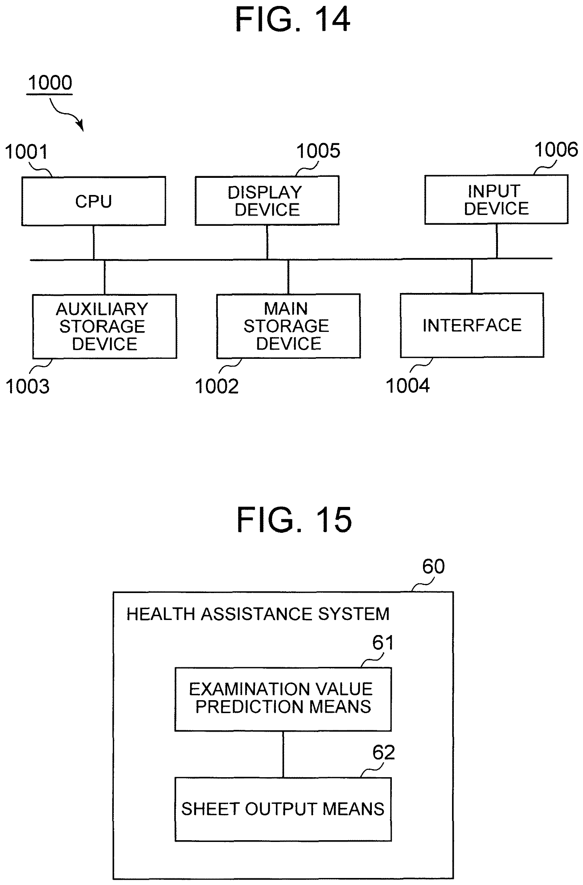

In addition, FIG. 14 is a schematic block diagram showing a configuration example of a computer according to the exemplary embodiment of the present invention. A computer 1000 includes a CPU 1001, a main storage device 1002, an auxiliary storage device 1003, an interface 1004, a display device 1005, and an input device 1006.

The health assistance system of the above-described exemplary embodiment may be mounted on the computer 1000. In this case, operation of the device may be stored in the auxiliary storage device 1003 in a form of a program. The CPU 1001 reads out a program from the auxiliary storage device 1003, expands the program in the main storage device 1002, and executes a predetermined process in the exemplary embodiment according to the program. Note that the CPU 1001 is an example of an information processing device that operates according to a program, and may include, in addition to a central processing unit (CPU), for example, a micro processing unit (MPU), a memory control unit (MCU), or graphics processing unit (GPU).

The auxiliary storage device 1003 is an example of a non-transitory tangible medium. Other examples of the non-transitory tangible medium include a magnetic disk, a magneto-optical disk, a CD-ROM, a DVD-ROM, and a semiconductor memory, connected via the interface 1004. In addition, in a case where this program is distributed to the computer 1000 via a communication line, the computer 1000 to which the program is distributed may expand the program in the main storage device 1002 and execute a predetermined process in each exemplary embodiment.

In addition, the program may be for implementing a part of a predetermined process in the exemplary embodiment. Furthermore, the program may be a difference program that implements a predetermined process in the exemplary embodiment in combination with another program already stored in the auxiliary storage device 1003.

The interface 1004 transmits/receives information to/from another device. In addition, the display device 1005 also presents information to a user. In addition, the input device 1006 receives input of information from a user.

In addition, depending on the processing contents in the exemplary embodiment, some elements of the computer 1000 can be omitted. For example, if the computer 1000 does not present information to a user, the display device 1005 can be omitted. For example, if the computer 1000 does not receive information input from a user, the input device 1006 can be omitted.

In addition, some or all of the above components are implemented by a general-purpose or dedicated circuitry, a processor, or a combination thereof. These components may be constituted by a single chip or a plurality of chips connected to each other via a bus. In addition, some or all of the above components may be achieved by a combination of the above-described circuitry and the like and a program.

In a case where some or all of the above components are achieved by a plurality of information processing devices, circuitries, and the like, the plurality of information processing devices, circuitries, and the like may be arranged in a concentrated manner or a distributed manner. For example, the information processing device, the circuitry, and the like may be achieved as a form in which a client and server system, a cloud computing system, and the like are connected to each other via a communication network.

Next, an outline of the present invention will be described. FIG. 15 is a block diagram showing an outline of the health assistance system of the present invention. A health assistance system 60 shown in FIG. 15 includes an examination value prediction means 61 and a sheet output means 62.

The examination value prediction means 61 (for example, the examination value prediction unit 12) predicts a future examination value for a prediction subject who is a designated person or a predetermined person using a prediction model specifying the relationship between a plurality of variables corresponding to one or more items relating to the current or past lifestyle habits of a certain person and a future examination value of the person.

The sheet output means 62 (for example, the sheet output unit 13) generates and outputs, on the basis of the result of prediction, an information providing sheet for each subject being assisted who is considered the subject of assistance among prediction subjects, the information providing sheet including information for improving the lifestyle habits, preventing disorders, or promoting health of the subject being assisted.

With such a configuration, it is possible to appropriately assist many people in changing their behaviors in order to prevent their disorders and promote their health.

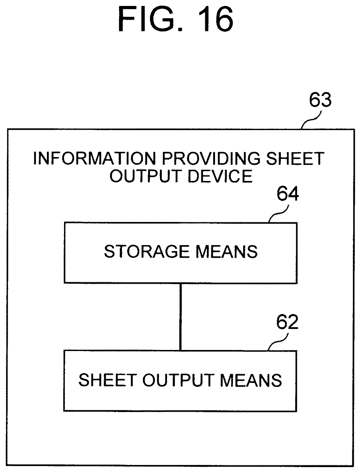

In addition, FIG. 16 is a block diagram showing an outline of the information providing sheet output device of the present invention. An information providing sheet output device 63 shown in FIG. 16 includes the above sheet output means 62 and a storage means 64.

The storage means 64 (for example, the data storage unit 14) stores prediction result data indicating a result of predicting a future examination value for a prediction subject who is a designated person or a predetermined person using a prediction model specifying the relationship between a plurality of variables corresponding to one or more items relating to the current or past lifestyle habits of a certain person and a future examination value of the person.

Even with such a configuration, it is possible to appropriately assist many people in changing their behaviors in order to prevent their disorders and promote their health.

Note that the above exemplary embodiment can be described as the following supplementary notes.
(Supplementary note 1) A health assistance system characterized by including: an examination value prediction means which predicts a future examination value for a prediction subject who is a designated person or a predetermined person using a prediction model specifying the relationship between a plurality of variables corresponding to one or more items relating to the current or past lifestyle habits of a certain person and a future examination value of the person; and a sheet output means which generates and outputs, on the basis of the result of prediction, an information providing sheet for each subject being assisted who is considered the subject of assistance among prediction subjects, the information providing sheet including information for improving the lifestyle habits, preventing disorders, or promoting health of the subject being assisted.
(Supplementary note 2) The health assistance system according to supplementary note 1, in which the prediction model is a prediction model specifying the relationship between a plurality of variables corresponding to examination values of a certain person for a past predetermined period and one or more items relating to current or past lifestyle habits, and a future examination value of the person, the examination value prediction means acquires at least the examination values for the past predetermined period used for the prediction model from record data of a prediction subject, and predicts a future examination value of the prediction subject, and in a case where there is a deficiency in the examination values for the past predetermined period used for the prediction model in the record data of the prediction subject, the examination value prediction means complements the deficient part with an examination value of another person in the same generation with the same sex as the prediction subject.
(Supplementary note 3) The health assistance system according to supplementary note 1 or 2, in which the examination value prediction means predicts, using a plurality of prediction models respectively corresponding to a plurality of examination items, respective future examination values of the plurality of examination items of a prediction subject, the examination value prediction means predicts a first examination value that is a future examination value of the prediction subject in a case where the prediction subject continues current lifestyle habits, and in a case where there is an examination value predicted to deteriorate in the future, for a high-risk examination item that is an examination item of the examination value, the examination value prediction means further predicts a second examination value that is a future examination value of the prediction subject in a case where the lifestyle habits are reviewed, and the information providing sheet includes the first examination value of each of the plurality of examination items of an subject being assisted, and in a case where the high-risk examination item is included in a prediction result of the subject being assisted, for a review item having a high improvement effect with respect to the high-risk examination item, the information providing sheet includes at least contents of the item after review and the second examination value after review.
(Supplementary note 4) The health assistance system according to supplementary note 3, in which the number of the high-risk examination items is two or less, and the sheet output means compares a current examination value with a future examination value, and selects the high-risk examination items in descending order of a deterioration ratio.
(Supplementary note 5) The health assistance system according to supplementary note 3 or 4, in which the examination value prediction means predicts, for the high-risk examination item, future examination values of all review patterns for one or more items relating to the lifestyle habits, and the sheet output means specifies a predetermined number of review patterns from all the review patterns in descending order of the degree of improvement, and uses an item changed in the specified review patterns as a review item with a high improvement effect with respect to the high-risk examination item.
(Supplementary note 6) The health assistance system according to any one of supplementary notes 1 to 5, in which the subject being assisted or a prediction subject who is a candidate for the subject being assisted is determined on the basis of a medical checkup ratio for a predetermined number of times of past predetermined medical checkups regularly performed.
(Supplementary note 7) The health assistance system according to any one of supplementary notes 1 to 6, in which the information providing sheet includes information indicating a specific means for improving lifestyle habits promoted by an organization to which a subject being assisted belongs.
(Supplementary note 8) The health assistance system according to any one of supplementary notes 1 to 7, in which in the information providing sheet, an address and a name of a subject being assisted are described at a position matched with a window frame of a predetermined envelope with a window.
(Supplementary note 9) The health assistance system according to any one of supplementary notes 1 to 8, in which the examination value prediction means predicts, using a plurality of prediction models respectively corresponding to a plurality of examination items, respective future examination values of the plurality of examination items of each of a plurality of prediction subjects, and when the sheet output means generates one information providing sheet for each subject being assisted who has been determined to be a target person of assistance among the plurality of prediction subjects, the sheet output means generates the information providing sheet as a file in which the information providing sheet of one subject being assisted is arranged on one page.
(Supplementary note 10) An information providing sheet output device characterized by including: a storage means which stores prediction result data indicating a result of predicting a future examination value for a prediction subject who is a designated person or a predetermined person using a prediction model specifying the relationship between a plurality of variables corresponding to one or more items relating to the current or past lifestyle habits of a certain person and a future examination value of the person; and a sheet output means which generates and outputs, on the basis of the result of prediction, an information providing sheet for each subject being assisted who is considered the subject of assistance among prediction subjects, the information providing sheet including information for improving the lifestyle habits, preventing disorders, or promoting health of the subject being assisted.
(Supplementary note 11) A health assistance method characterized by predicting a future examination value for a prediction subject who is a designated person or a predetermined person using a prediction model specifying the relationship between a plurality of variables corresponding to one or more items relating to the current or past lifestyle habits of a certain person and a future examination value of the person, and generating and outputting, on the basis of the result of prediction, an information providing sheet for each subject being assisted who is considered the subject of assistance among prediction subjects, the information providing sheet including information for improving the lifestyle habits, preventing disorders, or promoting health of the subject being assisted.
(Supplementary note 12) A health assistance program for causing a computer to execute: a process of predicting a future examination value for a prediction subject who is a designated person or a predetermined person using a prediction model specifying the relationship between a plurality of variables corresponding to one or more items relating to the current or past lifestyle habits of a certain person and a future examination value of the person; and a process of generating and outputting, on the basis of the result of prediction, an information providing sheet for each subject being assisted who is considered the subject of assistance among prediction subjects, the information providing sheet including information for improving the lifestyle habits, preventing disorders, or promoting health of the subject being assisted.

Hereinabove, the invention of the present application has been described with reference to the present exemplary embodiment and examples, but the present invention is not limited to the above exemplary embodiment and examples. Various modifications that can be understood by a person skilled in the art can be made to the configuration and details of the invention of the present application within the scope of the invention of the present application.

This application claims a priority based on Japanese Patent Application No. 2018-058535 that was filed on March 26, 2018, and incorporates the entire disclosure thereof herein.

### Industrial Applicability

The present invention can be suitably applied to health assistance in organizational units.

### Reference Signs List

- 100: Health assistance system
- 11: Model learning unit
- 12: Examination value prediction unit
- 13: Sheet output unit
- 14: Data storage unit
- 15: Target selection unit
- 1000: Computer
- 1001: CPU
- 1002: Main storage device
- 1003: Auxiliary storage device
- 1004: Interface
- 1005: Display device
- 1006: Input device
- 60: Health assistance system
- 61: Examination value prediction means
- 62: Sheet output means
- 63: Information providing sheet output device
- 64: Storage means

## Claims

1. A health assistance system comprising:
an examination value prediction means which predicts a future examination value for a prediction subject who is a designated person or a predetermined person using a prediction model specifying the relationship between a plurality of variables corresponding to one or more items relating to the current or past lifestyle habits of a certain person and a future examination value of the person; and
a sheet output means which generates and outputs, on the basis of the result of prediction, an information providing sheet for each subject being assisted who is considered the subject of assistance among prediction subjects, the information providing sheet including information for improving the lifestyle habits, preventing disorders, or promoting health of the subject being assisted.

2. The health assistance system according to claim 1, wherein
the prediction model is a prediction model specifying the relationship between a plurality of variables corresponding to examination values of a certain person for a past predetermined period and one or more items relating to current or past lifestyle habits, and a future examination value of the person,
the examination value prediction means acquires at least the examination values for the past predetermined period used for the prediction model from record data of a prediction subject, and predicts a future examination value of the prediction subject, and
in a case where there is a deficiency in the examination values for the past predetermined period used for the prediction model in the record data of the prediction subject, the examination value prediction means complements the deficient part with an examination value of another person in the same generation with the same sex as the prediction subject.

3. The health assistance system according to claim 1 or 2, wherein
the examination value prediction means predicts, using a plurality of prediction models respectively corresponding to a plurality of examination items, respective future examination values of the plurality of examination items of a prediction subject,
the examination value prediction means predicts a first examination value that is a future examination value of the prediction subject in a case where the prediction subject continues current lifestyle habits, and in a case where there is an examination value predicted to deteriorate in the future, for a high-risk examination item that is an examination item of the examination value, the examination value prediction means further predicts a second examination value that is a future examination value of the prediction subject in a case where the lifestyle habits are reviewed, and
the information providing sheet includes the first examination value of each of the plurality of examination items of an subject being assisted, and in a case where the high-risk examination item is included in a prediction result of the subject being assisted, for a review item having a high improvement effect with respect to the high-risk examination item, the information providing sheet includes at least contents of the item after review and the second examination value after review.

4. The health assistance system according to claim 3, wherein
the number of the high-risk examination items is two or less, and
the sheet output means compares a current examination value with a future examination value, and selects the high-risk examination items in descending order of a deterioration ratio.

5. The health assistance system according to claim 3 or 4, wherein
the examination value prediction means predicts, for the high-risk examination item, future examination values of all review patterns for one or more items relating to the lifestyle habits, and
the sheet output means specifies a predetermined number of review patterns from all the review patterns in descending order of the degree of improvement, and uses an item changed in the specified review patterns as a review item with a high improvement effect with respect to the high-risk examination item.

6. The health assistance system according to any one of claims 1 to 5, wherein
the subject being assisted or a prediction subject who is a candidate for the subject being assisted is determined on the basis of a medical checkup ratio for a predetermined number of times of past predetermined medical checkups regularly performed.

7. The health assistance system according to any one of claims 1 to 6, wherein
the information providing sheet includes information indicating a specific means for improving lifestyle habits promoted by an organization to which a subject being assisted belongs.

8. The health assistance system according to any one of claims 1 to 7, wherein
in the information providing sheet, an address and a name of a subject being assisted are described at a position matched with a window frame of a predetermined envelope with a window.

9. The health assistance system according to any one of claims 1 to 8, wherein
the examination value prediction means predicts, using a plurality of prediction models respectively corresponding to a plurality of examination items, respective future examination values of the plurality of examination items of each of a plurality of prediction subjects, and
when the sheet output means generates one information providing sheet for each subject being assisted who has been determined to be a target person of assistance among the plurality of prediction subjects, the sheet output means generates the information providing sheet as a file in which the information providing sheet of one subject being assisted is arranged on one page.

10. An information providing sheet output device comprising:
a storage means which stores prediction result data indicating a result of predicting a future examination value for a prediction subject who is a designated person or a predetermined person using a prediction model specifying the relationship between a plurality of variables corresponding to one or more items relating to the current or past lifestyle habits of a certain person and a future examination value of the person; and
a sheet output means which generates and outputs, on the basis of the result of prediction, an information providing sheet for each subject being assisted who is considered the subject of assistance among prediction subjects, the information providing sheet including information for improving the lifestyle habits, preventing disorders, or promoting health of the subject being assisted.

11. A health assistance method comprising:
predicting a future examination value for a prediction subject who is a designated person or a predetermined person using a prediction model specifying the relationship between a plurality of variables corresponding to one or more items relating to the current or past lifestyle habits of a certain person and a future examination value of the person; and
generating and outputting, on the basis of the result of prediction, an information providing sheet for each subject being assisted who is considered the subject of assistance among prediction subjects, the information providing sheet including information for improving the lifestyle habits, preventing disorders, or promoting health of the subject being assisted.

12. A health assistance program for causing a computer to execute:
a process of predicting a future examination value for a prediction subject who is a designated person or a predetermined person using a prediction model specifying the relationship between a plurality of variables corresponding to one or more items relating to the current or past lifestyle habits of a certain person and a future examination value of the person; and
a process of generating and outputting, on the basis of the result of prediction, an information providing sheet for each subject being assisted who is considered the subject of assistance among prediction subjects, the information providing sheet including information for improving the lifestyle habits, preventing disorders, or promoting health of the subject being assisted.
